# EUROPÄISCHE PATENTSCHRIFT

(11) **EP 3 255 035 B1**
(45) Veröffentlichungstag und Bekanntmachung des Hinweises auf die Patenterteilung: **19.09.2018**
(21) Anmeldenummer: 16173854.7
(22) Anmeldetag: 10.06.2016
(51) Int. Cl.: C07C 211/18, C08G 59/50

(54) **2-(3,3,5-TRIMETHYLCYCLOHEXYL)PROPAN-1,3-DIAMIN, EIN VERFAHREN ZU DESSEN HERSTELLUNG UND VERWENDUNG**
2- (3,3,5-TRIMETHYLCYCLOHEXYL) PROPANE-1,3-DIAMINE, A METHOD FOR THEIR PREPARATION AND USE
2-(3,3,5-TRIMETHYLCYCLOHEXYL) PROPANE-1,3-DIAMINE, PROCEDE DE FABRICATION ET D'UTILISATION

(43) Veröffentlichungstag der Anmeldung: 13.12.2017
(73) Patentinhaber: Evonik Degussa GmbH, 45128 Essen (DE)
(72) Erfinder: RÜFER, Alexander Martin, 45657 Recklinghausen (DE); RITTSTEIGER, Anne, 59399 Olfen (DE); NITZ, Jörg-Joachim, 45257 Essen (DE); KOHLSTRUK, Stephan, 45966 Gladbeck (DE); ORTELT, Martina, 74223 Flein (DE); FUCHSMANN, Dirk, 45721 Haltern am See (DE); DEMMING, Michael, 48249 Dülmen (DE); STEMMER, Christine, 45772 Marl (DE); OTT, Denise, 45772 Marl (DE); STASCH, Anja, 45657 Recklinghausen (DE)

(56) Entgegenhaltungen:
- EP-A2- 0 564 818
- WO-A1-2016/023837

## Beschreibung

Gegenstand der Erfindung ist ein neues Diamin mit der Bezeichnung 2-(3,3,5-trimethylcyclohexyl)propan-1,3-diamin, nachfolgend CPDA genannt, ein Verfahren zur Herstellung und Verwendung.
2-(3,3,5-trimethylcyclohexyl)propan-1,3-diamin (CPDA) hat die chemische Struktur wie in Formel 1 dargestellt. 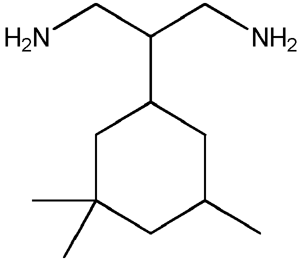 Formel 1: 2-(3,3,5-trimethylcyclohexyl)propan-1,3-diamin (CPDA)
Es ist bekannt, dass Diamine als Härter in Epoxidsystemen eingesetzt werden können. (Siehe zum Beispiel EP-A-0564818 und WO2016023837). Epoxidharze sind Prepolymere, welche pro Molekül zwei oder mehr Epoxidgruppen enthalten. Die Reaktion dieser Harze mit einer Reihe von Härtungsmitteln führt zu vernetzten Polymeren. Ein Überblick über mögliche Harze und Härter sowie deren Verwendung und Eigenschaften wird in H. Schumann, "Handbuch Betonschutz durch Beschichtung", Expert Verlag 1992, Seiten 396-428 gegeben.

Aufgabe war es, ein neues Diamin zu finden, welches für die Härtung von Epoxidsystemen geeignet ist.
Aufgabe war es, ein Verfahren zur Herstellung von 2-(3,3,5-trimethylcyclohexyl)propan-1,3-diamin (CPDA) zu finden.
Gegenstand der Erfindung ist das Diamin 2-(3,3,5-trimethylcyclohexyl)propan-1,3-diamin (CPDA) gemäß der Formel 1.
Gegenstand der Erfindung ist ein Verfahren zur Herstellung von 2-(3,3,5-trimethylcyclohexyl)propan-1,3-diamin, durch
A) Umsetzung von Isophoron (IP) und Malononitril zum Zwischenprodukt 2-(3,5,5-trimethylcyclohex-2-en-1-yliden)malononitril,
   und
B) Hydrierung von 2-(3,5,5-trimethylcyclohex-2-en-1-yliden)malononitril in Gegenwart mindestens eines Katalysators.

Die Herstellung der erfindungsgemäßen Verbindung erfolgt im ersten Schritt A) über eine Knoevenagelkondensation zwischen Isophoron (IP) und Malononitril. Die Reaktion kann im Lösungsmittel oder in einem lösungsmittelfreien Reaktionssystem unter milden Reaktionsbedingungen durchgeführt werden, bevorzugt bei 20-40°C und Normaldruck. Als Katalysator wird bevorzugt Zirkonylchlorid oder Piperidin eingesetzt. Nach vollständiger Umsetzung der Reaktanden kann das Zwischenprodukt 2-(3,5,5-trimethylcyclohex-2-en-1-yliden)malononitril (vgl. Struktur in Formel 2) durch Abkühlen der Reaktionslösung als Feststoff ausgefällt werden. Eine weitere Aufreinigung kann z.B. durch Destillation erfolgen.

Die Herstellung von CPDA aus 2-(3,5,5-trimethylcyclohex-2-en-1-yliden)malononitril erfolgt im Schritt B) über Hydrierung, die in einer oder mehreren Stufen durchgeführt werden kann. Bei der Verwendung von mehreren Hydrierungsreaktionen können die einzelnen Stufen in einem Reaktor mit unterschiedlichen Katalysatorzonen oder in mehreren separaten oder in Reihe geschalteten Reaktoren durchgeführt werden.

Bevorzugt erfolgt die Hydrierung in Festbettreaktoren. Geeignete Reaktortypen sind z.B. Schachtöfen, Hordenreaktoren oder Rohrbündelreaktoren. Es ist auch möglich, für die Hydrierung mehrere Festbettreaktoren hintereinander zu schalten, wobei jeder der Reaktoren wahlweise in Rieselbett- und Sumpffahrweise betrieben wird.

Als Katalysatoren können prinzipiell alle Katalysatoren eingesetzt werden, die die Hydrierung von Nitrilgruppen mit Wasserstoff katalysieren. Besonders geeignet sind Nickel-, Kupfer-, Eisen-, Palladium-, Rhodium-, Ruthenium- und Cobaltkatalysatoren, ganz besonders Palladium-Ruthenium- und Cobaltkatalysatoren. Zur Erhöhung der Aktivität, Selektivität und/oder Standzeit können die Katalysatoren zusätzliche Dotiermetalle oder andere Modifizierungsmittel enthalten. Typische Dotiermetalle sind z.B. Mo, Fe, Ag, Cr, Ni, V, Ga, In, Bi, Ti, Zr und Mn sowie die seltenen Erden. Typische Modifizierungsmittel sind z.B. solche, mit denen die Säure-Base-Eigenschaften der Katalysatoren beeinflusst werden können, bevorzugt Alkali- und Erdalkalimetalle bzw. deren Verbindungen, bevorzugt Mg- und Ca-Verbindungen, sowie Phosphorsäure oder Schwefelsäure sowie deren Verbindungen.
Die Katalysatoren können in Form von Pulvern oder Formkörpern, wie z.B. Extrudaten oder gepressten Pulvern, eingesetzt werden. Es können Vollkontakte, Raney-Typ-Katalysatoren oder Trägerkatalysatoren zur Anwendung kommen. Bevorzugt sind Raney-Typ- und Trägerkatalysatoren. Geeignete Trägermaterialien sind z.B. Siliciumdioxid, Aluminiumoxid, Alumosilikate, Titandioxid, Zirkoniumdioxid, Kieselgur, Aluminium-Silicium-Mischoxide, Magnesiumoxid und Aktivkohle. Das Aktivmetall kann in einer dem Fachmann bekannten Weise auf das Trägermaterial aufgebracht werden, wie z.B. durch Imprägnierung, Aufsprühen oder Fällung. Je nach Art der Katalysatorherstellung sind weitere, dem Fachmann bekannte, Präparationsschritte notwendig, wie z.B. Trocknung, Calcinierung, Formgebung und Aktivierung. Zur Formgebung können optional weitere Hilfsstoffe wie z.B. Graphit oder Magnesiumstearat zugesetzt werden. Das erforderliche Volumen der einzusetzenden Hydrierkatalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung zu gewährleisten.

Für die Herstellung des erfindungsgemäßen Diamins CPDA werden bevorzugt Hydrierkatalysatoren auf der Basis von Palladium und/oder Cobalt eingesetzt. Diese Katalysatoren weisen eine besonders gute Aktivität zur Erreichung einer hohen Ausbeute auf.
Die Katalysatoren können in Form von Pulvern oder Festbettkatalysatoren eingesetzt werden. Die Hydrierung kann in Batch-Fahrweise oder in kontinuierlich betriebenen Anlagen erfolgen.
Die Reaktionsbedingungen für die Hydrierung liegen zwischen 50-120°C und 20-300bar.

Die Hydrierung kann in ein oder mehreren Stufen durchgeführt werden. Bevorzugt wird die Hydrierung zweistufig durchgeführt. Hierbei werden in der ersten Stufe Reaktionsbedingungen von 20-120°C und 20-300 bar, bevorzugt 40-100°C und 25-150 bar und besonders bevorzugt 60-90°C und 40-80 bar gewählt. In der zweiten Stufe der Hydrierung werden Reaktionsbedingungen von 20-120°C und 20-300 bar, bevorzugt 50-115°C und 50-200 bar und besonders bevorzugt 80-110°C und 80-140 bar gewählt.
In der ersten Stufe der Hydrierung wird bevorzugt ein Palladium-Katalysator eingesetzt.

In der zweiten Stufe der Hydrierung wird bevorzugt ein Raney-Typ-Katalysator eingesetzt. Der Katalysator weist besonders bevorzugt nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

und
mit Partikelgrößen des Katalysators, also der Granulat-Teilchen mit einer statistischen Verteilung zwischen 3 bis 7 Millimetern (mm), wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

Das die Hydrierung verlassende Reaktionsgemisch wird mit den üblichen Methoden weiter aufgereinigt, um ein CPDA mit der gewünschten Qualität zu erhalten. Dabei können alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich, absatzweise, ein- oder mehrstufig, im Vakuum oder unter Druck durchgeführt werden.

Bevorzugt wird die Aufreinigung durch Destillation unter Druck und/oder im Vakuum in mehreren Schritten erreicht. Hierfür lassen sich beliebige Destillationskolonnen mit und ohne Einbauten wie z.B. Dephlegmatoren, Trennwänden, ungeordnete Einbauten oder Schüttungen, geordnete Einbauten oder Packungen, Böden mit oder ohne Zwangsführung verwenden. Die Aufreinigung von CPDA wird bevorzugt durch Destillation durchgeführt.

### Anwendung als Epoxidhärter

Gegenstand der Erfindung ist auch die Verwendung von 2-(3,3,5-trimethylcyclohexyl)propan-1,3-diamin (CPDA) als Härter in Epoxidharzzusammmensetzungen.

Als Epoxidharzkomponente kommen prinzipiell alle Epoxidharze in Frage, die mit Aminen gehärtet werden können. Zu den Epoxidharzen zählen z.B. Polyepoxide auf Basis von Bisphenol A-diglycidylether, Bisphenol F-diglycidylether oder cycloaliphatische Typen. Vorzugsweise werden jedoch Epoxidharze auf Basis Bisphenol A und gegebenenfalls die auf Basis Bisphenol F - ggf. auch in Mischung - eingesetzt. Harze und Härter werden dabei bevorzugt in äquivalenten Mengen eingesetzt. Es sind jedoch auch Abweichungen vom stöchiometrischen Verhältnis möglich.

### Beispiele

### Beispiel 1: Herstellung von 2-(3,3,5-trimethylcyclohexyl)propan-1,3-diamin (CPDA)

### Schritt A): Synthese von 2-(3,5,5-trimethylcyclohex-2-en-1-yliden)malononitril.

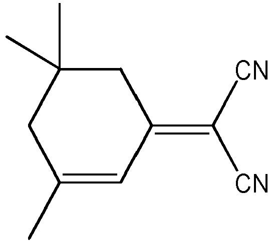

Formel 2: 2-(3,5,5-trimethylcyclohex-2-en-1-yliden)malononitril
- In einen 2 L-Dreihalskolben mit zwei Tropftrichtern wurde 484 g Isophoron (IP) vorgelegt. Der Reaktorinhalt wurde dabei auf Raumtemperatur gehalten.
- 231 g Malononitril wurden mit 250 g Ethanol (EtOH) verdünnt und in einem Tropftrichter vorgelegt.
- 7 g Piperidin (Katalysator) wurden mit 50 g EtOH verdünnt und in den zweiten Tropftrichter gefüllt.
- Anschließend wurde der Inhalt beider Tropftrichter simultan tropfenweise in den Reaktor gegeben und der Reaktor im Anschluss für zwei Stunden bei 50°C gerührt.
- Die entstandene Reaktionsmischung wurde auf 10°C gekühlt und das dabei ausgefallende Produkt (2-(3,5,5-trimethylcyclohex-2-en-1-yliden)malononitril) abfiltriert.
- Die weitere Aufreinigung erfolgte über eine Umkristallisation in kaltem Ethanol und einer anschließenden Filtration und Trocknung im Vakuumtrockenschrank (45°C, 10 mbar, 3 h).
- Die Produktzusammensetzung wurde mittels Gaschromatographie ermittelt.

Die Ausbeute betrug 50 % an 2-(3,5,5-trimethylcyclohex-2-en-1-yliden)malononitril.

### Schritt B1): Teilhydrierung von 2-(3,5,5-trimethylcyclohex-2-en-1-yliden)malononitril zur Herstellung von CPDA, 1. Hydrierstufe.

- In einen 2 L-Druckautoklaven mit Katalysatorkorb wurden 150 ml des Festbettkatalysators Pd/Aluminiumoxid (1 Gew.-% Pd) eingebaut.
- Für die Reaktion wurde 1 L Lösung mit 10 Gew.-% 2-(3,5,5-trimethylcyclohex-2-en-1-yliden)malononitril (Produkt aus Schritt A) in Tetrahydrofuran vorgelegt.
- Die Reaktion erfolgte bei 75°C, 50 bar Wasserstoff für 5 h.
- Die komplette Produktlösung wurde aus dem Reaktor.
- Die Zusammensetzung der Produktlösung wurde mittels Gaschromatographie bestimmt.

### Schritt B2) 2. Hydrierstufe: Vollhydrierung von Produktlösung aus Schritt B.

- In einen 2 L-Druckautoklaven mit Katalysatorkorb wurden 150 ml aktivierte Cobalt-Legierungsgranulate nach Raney als Festbett eingebaut. Der Katalysator wies dabei die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 75,9 Gew.-% |
| Aluminium: | 20,0 Gew.-% |
| Chrom: | 1,5 Gew.-% |
| Nickel: | 2,6 Gew.-% |

Es wurde eine Siebfraktion des Katalysators mit einer statistischen Verteilung zwischen 2,0 und 5,0 Millimetern (mm) eingesetzt, wobei bis zu 10% der Partikel oberhalb der genannten Obergrenze und bis zu 10% der Partikel unterhalb der genannten Untergrenze liegen können.
- Für die Reaktion wurde 1 L Reaktionslösung (teilhydriertes Produkt aus Schritt B1 in THF) vorgelegt.
- Die Reaktion erfolgte bei 100°C, 100 bar Wasserstoff für 5 h.
- Die Zusammensetzung der Produktlösung wurde mittels Gaschromatographie ermittelt.
- Für den Einsatz von CPDA als Härter in Epoxidharzsystemen wurde das erhaltene Produkt mittels Destillation gereinigt.

Die Ausbeute der zweistufigen Hydrierung (Schritt B1 und B2) betrug 83 Gew.-% CPDA bezogen auf das eingesetzte Dinitril aus Stufe A.

### Beispiel 2: CPDA als Härter in Epoxidharzsystemen

Als Epoxidharz wurde das Standardharz Epikote 828 der Firma Hexion mit EpoxidÄquivalentgewicht 188 g/val verwendet. Dieses wurde in stöchiometrischer Übereinstimmung der H-Äquivalente mit der Härterkomponente CPDA vermengt (vgl. Tabelle 1) und die Glasübergangstemperatur (Tg) nach einer Verweilzeit von einer Stunde bei definierter Härtungstemperatur bestimmt (Tabelle 2). Die jeweiligen Reaktionsumsätze wurden über die aufgenommene Wärmetönung der Härtungsreaktion in Relation zur Maximalwärmetönung bestimmt (Tabelle 3).

**Tabelle 1: Verhältnis von Harz zu Härter**

| | |
|---|---|
| Härterkomponente CPDA (g) | 100 |
| Menge Epoxidharz (g) pro 100 g Härter | 380 |

**Tabelle 2: Glasübergangstemperaturen (Tg) nach 1 h Härtung bei verschiedenen Temperaturen**

| | |
|---|---|
| Tgmax. (DSC) | 135°C |
| Tg nach 1 h 50°C | 47°C |
| Tg nach 1 h 70°C | 75°C |
| Tg nach 1 h 90°C | 97°C |
| Tg nach 1 h 110°C | 117°C |
| Tg nach 1 h 130°C | 130°C |
| Tg nach 1 h 150°C | 134°C |

**Tabelle 3: Umsätze**

| | |
|---|---|
| Umsatz nach 1 h 50°C | 90 % |
| Umsatz nach 1 h 70°C | 90 % |
| Umsatz nach 1 h 90°C | 92 % |
| Umsatz nach 1 h 110°C | 100 % |
| Umsatz nach 1 h 130°C | 100 % |
| Umsatznach 1 h 150°C | 100 % |

Wie der Fachmann anhand Tabelle 1, Tabelle 2 und Tabelle 3 leicht erkennen kann, eignet sich CPDA als Härterkomponente in Epoxidharzsystemen.

## Patentansprüche

1. Diamin 2-(3,3,5-trimethylcyclohexyl)propan-1,3-diamin der Formel 1

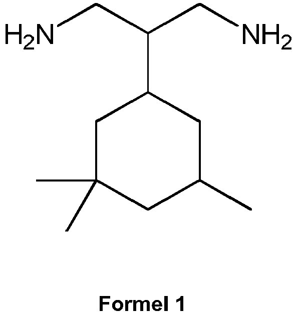

2. Verfahren zur Herstellung von 2-(3,3,5-trimethylcyclohexyl)propan-1,3-diamin, durch
A) Umsetzung von Isophoron (IP) und Malononitril zum Zwischenprodukt 2-(3,5,5-trimethylcyclohex-2-en-1-yliden)malononitril, und
B) Hydrierung von 2-(3,5,5-trimethylcyclohex-2-en-1-yliden)malononitril in Gegenwart mindestens eine Katalysators.

3. Verfahren nach Anspruch 2, wobei die Hydrierung im Schritt B) bei 20-120°C und bei 20-300 bar durchgeführt wird.

4. Verfahren nach Anspruch 2, wobei die Hydrierung im Schritt B) in zwei Stufen bei 20-120°C und bei 20-300 bar durchgeführt wird.

5. Verfahren nach Anspruch 4 wobei die Hydrierung in der ersten Stufe bei 40-100°C und 25-150 bar, und in der zweiten Stufe bei 50-115°C und 50-200 bar durchgeführt wird.

6. Verfahren nach Anspruch 4-5, wobei die Hydrierung in der ersten Stufe bei 60-90°C und 40-80 bar, und in der zweiten Stufe bei 80-110°C und 80-140 bar durchgeführt wird.

7. Verfahren nach mindestens einem der vorherigen Ansprüche, **dadurch gekennzeichnet, dass** Katalysatoren ausgewählt aus Nickel-, Kupfer-, Eisen-, Palladium-, Rhodium-, Ruthenium- und Cobaltkatalysatoren, eingesetzt werden.

8. Verfahren nach mindestens einem der vorherigen Ansprüche, **dadurch gekennzeichnet, dass** als Katalysatoren Palladium und/oder Cobaltkatalysatoren eingesetzt werden.

9. Verfahren nach mindestens einem der vorherigen Ansprüche, **dadurch gekennzeichnet, dass** als Katalysatoren Raney-Typ-Katalysatoren oder Trägerkatalysatoren eingesetzt werden.

10. Verfahren nach mindestens einem der vorherigen Ansprüche, **dadurch gekennzeichnet, dass** als Katalysator ein Katalysator aus aktivierten Cobalt-Legierungsgranulaten nach Raney eingesetzt wird, wobei der Katalysator nach der Aktivierung in seiner Gesamtheit die folgende Zusammensetzung in Gewichtsprozent (Gew.-%) aufweist, wobei sich die Anteile zu 100 Gew.-% addieren, bezogen auf die enthaltenden Metalle:
| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |
und
mit Partikelgrößen des Katalysators, also die Granulat-Teilchen mit einer statistischen Verteilung zwischen 3 bis 7 Millimeter (mm), wobei bis zu 10 Prozent der Partikel auch außerhalb des genannten Bereichs der genannten Untergrenze oder Obergrenze, liegen können.

11. Verfahren nach mindestens einem der vorherigen Ansprüche, **dadurch gekennzeichnet, dass** die Hydrierung in Festbettreaktoren, bevorzugt in Schachtöfen, Hordenreaktoren oder Rohrbündelreaktoren, durchgeführt wird.

12. Verwendung von 2-(3,3,5-trimethylcyclohexyl)propan-1,3-diamin als Härter in Epoxidharzzusammmensetzungen.

## Claims

1. Diamine 2-(3,3,5-trimethylcyclohexyl)propane-1,3-diamine of formula 1

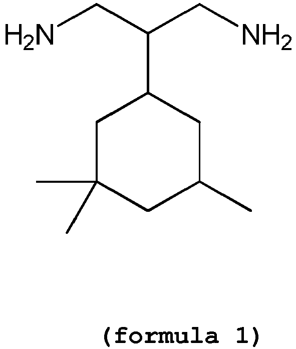

2. Process for producing 2-(3,3,5-trimethylcyclohexyl)propane-1,3-diamine by
A) reacting isophorone (IP) and malononitrile to afford the intermediate 2-(3,5,5-trimethylcyclohex-2-en-1-ylidene)malononitrile,
and
B) hydrogenating 2-(3,5,5-trimethylcyclohex-2-en-1-ylidene)malononitrile in the presence of at least one catalyst.

3. Process according to Claim 2, wherein the hydrogenation in step B) is performed at 20-120°C and at 20-300 bar.

4. Process according to Claim 2, wherein the hydrogenation in step B) is performed in two stages at 20-120°C and at 20-300 bar.

5. Process according to Claim 4, wherein the hydrogenation is performed at 40-100°C and 25-150 bar in the first stage and at 50-115°C and 50-200 bar in the second stage.

6. Process according to Claims 4-5, wherein the hydrogenation is performed at 60-90°C and 40-80 bar in the first stage and at 80-110°C and 80-140 bar in the second stage.

7. Process according to at least one of the preceding claims, **characterized in that** catalysts selected from nickel, copper, iron, palladium, rhodium, ruthenium and cobalt catalysts are employed.

8. Process according to at least one of the preceding claims, **characterized in that** the catalysts employed are palladium and/or cobalt catalysts.

9. Process according to at least one of the preceding claims, **characterized in that** the catalysts employed are Raney-type catalysts or supported catalysts.

10. Process according to at least one of the preceding claims, **characterized in that** the catalyst employed is a catalyst composed of activated Raney cobalt alloy pellets, wherein after activation the catalyst in its entirety has the following composition in weight per cent (wt%), the proportions summing to 100 wt% based on the metals present:
| | |
|---|---|
| cobalt: | 57 to 84 wt% |
| aluminium: | 10 to 40 wt% |
| chromium: | 1 to 2 wt% |
| nickel: | 2 to 4 wt% |
and
with particle sizes of the catalyst, i.e. the pellet particles, having a statistical distribution between 3 to 7 millimetres (mm), wherein up to 10 per cent of the particles may also be outside the stated range of the stated lower limit or upper limit.

11. Process according to at least one of the preceding claims, **characterized in that** the hydrogenation is performed in fixed-bed reactors, preferably in shaft furnaces, tray reactors or shell and tube reactors.

12. Use of 2-(3,3,5-trimethylcyclohexyl)propane-1,3-diamine (CPDA) as a hardener in epoxy resin compositions.

## Revendications

1. Diamine 2-(3,3,5-triméthylcyclohexyl)propane-1,3-diamine de formule 1

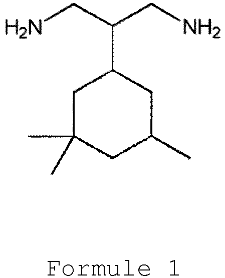

2. Procédé de fabrication de 2-(3,3,5-triméthylcyclohexyl)propane-1,3-diamine, par
A) mise en réaction d'isophorone (IP) et de malononitrile pour former le produit intermédiaire 2-(3,5,5-triméthylcyclohex-2-én-1-ylidène)malononitrile,
et
B) hydrogénation de 2-(3,5,5-triméthylcyclohex-2-én-1-ylidène)malononitrile en présence d'au moins un catalyseur.

3. Procédé selon la revendication 2, dans lequel l'hydrogénation à l'étape B) est réalisée à une température de 20 à 120 °C et une pression de 20 à 300 bar.

4. Procédé selon la revendication 2, dans lequel l'hydrogénation à l'étape B) est réalisée en deux étapes à une température de 20 à 120 °C et une pression de 20 à 300 bar.

5. Procédé selon la revendication 4, dans lequel l'hydrogénation est réalisée à la première étape à une température de 40 à 100 °C et une pression de 25 à 150 bar et à la deuxième étape à une température de 50 à 115 °C et une pression de 50 à 200 bar.

6. Procédé selon les revendications 4 à 5, dans lequel l'hydrogénation est réalisée à la première étape à une température de 60 à 90 °C et une pression de 40 à 80 bar et à la deuxième étape à une température de 80 à 110 °C et une pression de 80 à 140 bar.

7. Procédé selon au moins l'une quelconque des revendications précédentes, **caractérisé en ce que** des catalyseurs choisis parmi les catalyseurs à base de nickel, cuivre, fer, palladium, rhodium, ruthénium et cobalt sont utilisés.

8. Procédé selon au moins l'une quelconque des revendications précédentes, **caractérisé en ce que** des catalyseurs à base de palladium et/ou de cobalt sont utilisés en tant que catalyseurs.

9. Procédé selon au moins l'une quelconque des revendications précédentes, **caractérisé en ce que** des catalyseurs de type Raney ou des catalyseurs supportés sont utilisés en tant que catalyseurs.

10. Procédé selon au moins l'une quelconque des revendications précédentes, **caractérisé en ce qu'**un catalyseur à base de granulats d'un alliage de cobalt activé selon Raney est utilisé en tant que catalyseur, le catalyseur présentant dans son ensemble après l'activation la composition suivante en pourcentages en poids (% en poids), la somme des proportions étant de 100 % en poids, en termes des métaux contenus :
| | |
|---|---|
| cobalt : | 57 à 84 % en poids |
| aluminium : | 10 à 40 % en poids |
| chrome : | 1 à 2 % en poids |
| nickel : | 2 à 4 % en poids |
et
avec des tailles de particules du catalyseur, c'est-à-dire les particules du granulat, ayant une distribution statistique comprise entre 3 et 7 millimètres (mm), jusqu'à 10 pour cent des particules pouvant également se situer en dehors de la plage indiquée de la limite inférieure ou de la limite supérieure indiquée.

11. Procédé selon au moins l'une quelconque des revendications précédentes, **caractérisé en ce que** l'hydrogénation est réalisée dans des réacteurs à lit fixe, de préférence dans des fours verticaux, des réacteurs à étages ou des réacteurs à faisceau de tubes.

12. Utilisation de 2-(3,3,5-triméthylcyclohexyl)-propane-1,3-diamine en tant que durcisseur dans des compositions de résine époxyde.
